# EUROPÄISCHE PATENTSCHRIFT

(11) **EP 0 862 899 B1**
(45) Veröffentlichungstag und Bekanntmachung des Hinweises auf die Patenterteilung: **14.01.2004**
(21) Anmeldenummer: 98103749.2
(22) Anmeldetag: 03.03.1998
(51) Int. Cl.: A61C 1/08, A61B 5/00, A61C 1/18

(54) **Zahnärztliche Behandlungsvorrichtung**
Dental treatment device
Dispositif de traitement dentaire

(30) Priorität: 07.03.1997 DE 19709499
(43) Veröffentlichungstag der Anmeldung: 09.09.1998
(73) Patentinhaber: Kaltenbach & Voigt GmbH & Co. KG, 88400 Biberach/Riss (DE)
(72) Erfinder: Löhn, Gerd, 88400 Biberach-Rissegg (DE)
(74) Vertreter: Schmidt-Evers, Jürgen, Dipl.-Ing.

(56) Entgegenhaltungen:
- EP-A- 0 217 093
- DE-A- 3 339 650

## Beschreibung

Die vorliegende Erfindung betrifft eine zahnärztliche Behandlungsvorrichtung, insbesondere eine zahnärztliche Behandlungsvorrichtung zum Erkennen von Karies, Plaque oder bakteriellem Befall an Zähnen.

Zahnärztliche Behandlungsvorrichtungen umfassen bekannterweise ein zahnärztliches Handstück, auf das einerseits ein zahnärztliches Behandlungsinstrument oder -werkzeug angebracht und andererseits ein Versorgungsschlauch angeschlossen werden kann, der wiederum mit einer Steuereinrichtung verbunden ist, die dem zahnärztlichen Handstück mit dem zahnärztlichen Behandlungsinstrument über den Versorgungsschlauch die für das zahnärztliche Behandlungsinstrument jeweils geeigneten Betriebsmittel, wie z.B. Strom, Kühlwasser, Kühlspray oder Licht, zuführt. Im Falle einer zahnärztlichen Behandlungsvorrichtung zum Erkennen von Karies, Plaque oder bakteriellem Befall an Zähnen handelt es sich bei dem zahnärztlichen Behandlungsinstrument um eine Lichtsonde, die den zu untersuchenden Zahn mit einer primären Lichtstrahlung bestrahlt und zugleich an dem bestrahlten Zahn angeregte Fluoreszenzstrahlung als sekundäre Lichtstrahlung detektiert und an die Steuereinrichtung weiterleitet. Die Steuereinrichtung schließt aufgrund der spektralen Eigenschaften der detektierten Sekundärstrahlung auf den kariösen Zustand des untersuchten Zahnes. Entsprechende Vorrichtungen sind beispielsweise in der US-A-4,479,499, DE-A1-42 00 741 oder DE-U1-93 17 984 beschrieben.

Bei den bekannten zahnärztlichen Behandlungsvorrichtungen wird das zahnärztliche Behandlungsinstrument entweder auf das zahnärztliche Handstück aufgeschraubt oder aufgesteckt. Im Falle des Aufschraubens ist zum Entfernen des zahnärztlichen Behandlungsinstrumentes dessen Abschrauben von dem zahnärztlichen Handstück notwendig. Im Falle des Aufsteckens wird das zahnärztliche Behandlungsinstrument durch Paßsitz auf dem zahnärztlichen Handstück fixiert, so daß zum Entfernen und Abziehen des zahnärztlichen Behandlungsinstruments ein relativ großer Kraftaufwand erforderlich ist. In der Regel wird auch der Versorgungsschlauch auf das Handstück aufgeschraubt oder aufgesteckt, so daß bezüglich der Kopplung zwischen dem Versorgungsschlauch und dem zahnärztlichen Handstück ebenfalls das zuvor Gesagte zutrifft.

Die europäische Offenlegungsschrift EP 0 217 093 beschreibt ein zahnärztliches Handstück, welches einerseits ein an einen Versorgungsschlauch anschließbares Griffteil und andererseits ein mit dem Griffteil koppelbares Kopfteil aufweist. Dabei weist das Griffteil eine schräg verlaufende Anlagefläche auf, die nach Aufstecken des Kopfteils auf das Griffteil an einer komplementär ausgebildeten Anlagefläche des Kopfteils anliegt. Um das Kopfteil mit dem Griffteil zu koppeln, wird ein Betätigungselement verwendet, welches eine ebenfalls. komplementär zu der Fläche des Kopfteils ausgebildete Anlagefläche aufweist. Mit Hilfe der komplementär ausgestalteten Anlageflächen wird eine Übertragung von Behandlungslicht über das Griffteil zu dem Kopfteil hin gewährleistet, so dass Licht ohne Schlagschatten und bei Vermeidung unnötiger Verluste an Trenn- bzw. Kupplungsstellen möglichst dicht an eine Präparationsstelle herangeführt werden kann.

Aus der DE 33 39 650 A1 ist ebenfalls eine Kupplungsvorrichtung zum axialen Verbinden von zwei Teilen eines zahnärztlichen Handstücks bekannt, wobei diese Schrift ausschließlich einen Kupplungsübergang innerhalb des zahnärztlichen Handstücks selbst betrifft, insbesondere den Kupplungsübergang zwischen einem Griffteil des zahnärztlichen Handstücks und einem Antriebsteil mit daran befestigtem Verbindungsschlauch. Dabei enthält das Antriebsteil ausgehend von einer Kante beidseitig schraubförmig verlaufende Gleitflächen, die bei Kupplung mit dem Griffteil formschlüssig mit den entsprechend ausgebildeten Gegenflächen des Griffteils in Eingriff gebracht werden. Nach Aufsetzen des Griffteils auf das Antriebsteil liegt eine im Antriebsteil ausgebildete Lichtaustrittsöffnung dem Ende eines im Griffteil ausgebildeten Lichtleiters gegenüber: An dem Antriebsteil sind zwei Rastkugeln vorgesehen, die bei Ineinanderstecken des Griffteils und des Antriebsteils in eine Ringnut des Griffteils einrasten, um somit das Griffteil gegen ein axiales Abgleiten zu sichern.

Der vorliegenden Erfindung liegt daher die Aufgabe zugrunde, eine zahnärztliche Behandlungsvorrichtung zu schaffen, bei der das Abnehmen bzw. Entfernen des zahnärztlichen Behandlungsinstruments von dem zahnärztlichen Handstück erleichtert ist.

Diese Aufgabe wird erfindungsgemäß durch eine zahnärztliche Behandlungsvorrichtung nach Anspruch 1 gelöst.

Gemäß der vorliegenden Erfindung sind die zu verbindenden Teile der zahnärztlichen Behandlungsvorrichtung jeweils mit komplementären Entriegelungsschrägen oder Entriegelungsebenen, die schräg zur. Längsrichtung des zahnärztlichen Handstücks verlaufen, ausgestattet, so daß zum Lösen der aufgesteckten Teile lediglich ein Drehen der miteinander gekoppelten Teile gegeneinander in Umfangsrichtung des zahnärztlichen Handstückes erforderlich ist. Dadurch, daß im aufgesteckten Zustand die Entriegelungsebenen der gekoppelten Teile aneinander anliegen, werden die gekoppelten Teile automatisch durch das Verdrehen in Umfangsrichtung des zahnärztlichen Handstücks voneinander in Längsrichtung des zahnärztlichen Handstücks entfernt, so daß ein Ziehen in Längsrichtung des zahnärztlichen Handstücks zum Lösen der Verbindung nicht mehr erforderlich ist. Auf diese Weise wird der Kraftaufwand zum Lösen der gekoppelten Teile deutlich verringert:

Die Unteransprüche beschreiben: vorteilhafte Ausführungsformen der vorliegenden Erfindung.

Die. Erfindung wird nachfolgend anhand bevorzugter Ausführungsbeispiele unter Bezugnahme auf die beigefügte Zeichnung näher erläutert. Dabei zeigt:
Fig.1 eine erfindungsgemäße zahnärztliche Behandlungsvorrichtung zum Erkennen von Karies, Plaque oder bakteriellem Befall an Zähnen,
Fig. 2a eine vergröBerte Darstellung der Kopplungsbereiche zwischen einem zahnärztlichen Behandlungsinstrument bzw. einem Versorgungsschlauch und einem zahnärztlichen Handstück bei der in Fig. 1 gezeigten zahnärztlichen Behandlungsvorrichtung,
Fig. 2b - d verschiedene Spitzenformen für die in Fig. 2a dargestellte Lichtsonde,
Fig. 3 eine vergröBerte Darstellung des erfindungsgemäßen zäünäiztlichen Handstücks,
Fig. 4a eine vergrößerte Ansicht einer erfindungsgemäßen Lichtsonde zum Untersuchen von glatten Zahnflächen,
Fig. 4b eine vergrößerte Ansicht einer erfindungsgemäßen Lichtsonde zur Untersuchung von Zwischenzahnbereichen (Approximalbereichen), und
Fig. 4c eine vergrößerte Darstellung einer erfindungsgemäßen Lichtsonde zur Untersuchung von Fissuren.

Die erfindungsgemäße zahnärztliche Behandlungsvorrichtung wird nachfolgend anhand einer zahnärztlichen Behandlungsvorrichtung zum Ermitteln von Karies, Plaque oder bakteriellem Befall an Zähnen beispielhaft erläutert. Selbstverständlich kann jedoch auch die Erfindung auf andere zahnärztliche Behandlungsvorrichtungen angewendet werden, bei denen anstelle einer Lichtsonde andere Behandlungsinstrumente oder Behandlungswerkzeuge, wie z.B. Bohrer, Sprayaufsätze oder andere Beleuchtungseinrichtungen, auf das zahnärztliche Handstück aufgesetzt werden können.

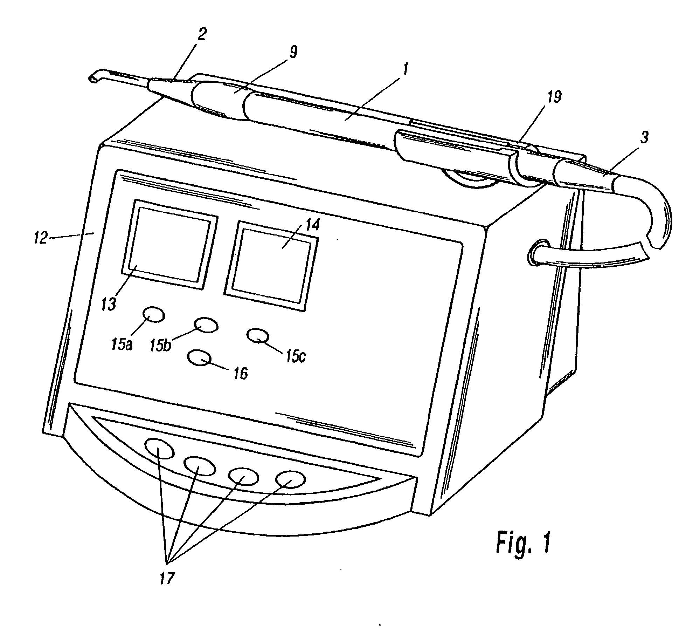

Fig. 1 zeigt die Gesamtdarstellung einer erfindungsgemäßen zahnärztlichen Behandlungsvorrichtung zum Ermitteln von Karies, Plaque oder bakteriellem Befall. Die in Fig. 1 dargestellte Behandlungsvorrichtung umfaßt eine Steuereinrichtung 12, ein über einen Versorgungsschlauch 3 mit der Steuereinrichtung verbundenes zahnärztliches Handstück 1 sowie ein mit dem zahnärztlichen Handstück 1 gekoppeltes zahnärztliches Behandlungsinstrument, in diesem Fall eine Lichtsonde 2. Die Lichtsonde 2 und/oder der Schlauchanschluß des Versorgungschlauches 3 sind auf das zahnärztliche Handstück 1 aufgesteckt. Mit Hilfe der in Fig. 1 gezeigten zahnärztlichen Behandlungsvorrichtung wird ein zu untersuchender Zahn mit Licht bestrahlt, wobei die von der Lichtsonde 2 abgegebene Primärstrahlung an dem zu untersuchenden Zahn eine Fluoreszenzstrahlung anregt. Das von dem Zahn zurückgestrahlte Fluoreszenzspektrum weist dabei deutliche Unterschiede zwischen einem gesunden (Zahn-) Gewebe und einem im Vergleich zum gesunden Zustand veränderten (kranken) Gewebe auf. Insbesondere treten deutliche Unterschiede zwischen kariösen und gesunden Zahnbereichen auf. Das Fluoreszenzspektrum wird von der Lichtsonde 2 detektiert und über eine Lichtleiter in dem zahnärztlichen Handstück 1 und dem Versorgungsschlauch 3 an die Steuereinrichtung 12 weitergeleitet, wo aufgrund der erfaßten Fluoreszenzstrahlungsintensität ein gesunder Zahnbereich eindeutig von einem kariösen Zahnbereich unterschieden werden kann. Das in Fig. 1 gezeigte Gerät weist eine Anzeige 13 zum Anzeigen des aktuellen Meßwerts der an dem bestrahlten Zahn angeregten Fluoreszenzstrahlung und eine Anzeige 14 zum Anzeigen eines maximalen Meßwerts (Spitzenwerts) der erfaßten Fluoreszenzstrahlung auf. Wie noch näher erläutert wird, wird erfindungsgemäß vorgeschlagen, für unterschiedliche Anwendungen bzw. zu untersuchende Zahnbereiche unterschiedlich geformte Lichtsonden zu verwenden. Aus diesem Grund sind Sondenanzeigen 15a-c vorgesehen, die durch Aufleuchten jeweils die gewählte und auf das zahnärztliche Handstück aufgesteckte Lichtsonde 2 anzeigen. Vorteilhafterweise wird die in Fig. 1 gezeigte Behandlungsvorrichtung mit einer Batterie oder einem Akkumulator als Spannungsquelle betrieben, so daß die Behandlungsvorrichtung mobil und leicht ist. Eine Warnlampe 16 weist durch Aufleuchten darauf hin, daß die von der Batterie bzw. dem Akkumulator abgegebene Spannung unter einen vorgegebenen Minimalspannungswert gesunken ist und somit ausgetauscht oder aufgeladen werden muß. Zudem sind Einstelltasten 17 vorgesehen, mit deren Hilfe u.a. eine gewählte und aufgesteckte Lichtsonde 2 kalibriert und der Typ der Lichtsonde 2 der Steuereinrichtung 12 mitgeteilt werden kann. Das zahnärztliche Handstück 1 kann, beispielsweise zum Transport, in einer Halterung 19 abgelegt werden. Vorteilhafterweise weist das zahnärztliche Handstück 1 an seinem instrumentenseitigen Ende einen Ringschalter 9 auf. Dieser Ringschalter 9 ist ein sich um das zahnärztliche Handstück 1 ringförmig erstreckender und auf Druck ansprechender Schalter, so daß über diesen Schalter unabhängig von der Lage des zahnärztlichen Handstücks 1 in der Hand einer Bedienperson die Behandlungsvorrichtung ein/ausgeschaltet oder ein bestimmter Betriebsmodus ausgewählt werden kann.

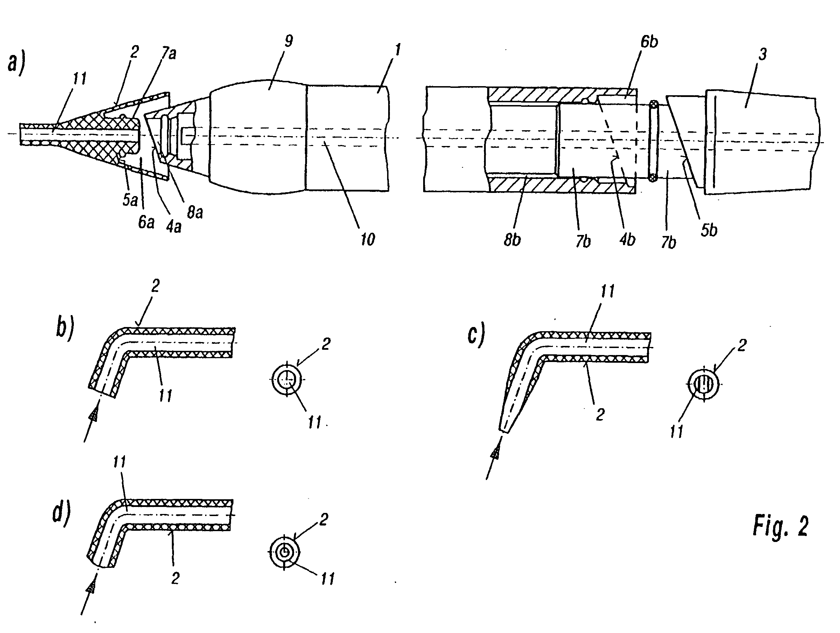

Fig. 2a zeigt eine vergrößerte Darstellung der erfindungsgemäßen Kopplungsmechanismen zwischen dem in Fig. 1 dargestellten zahnärztlichen Behandlungsinstrument 2 bzw. Versorgungsschlauch 3 und dem zahnärztlichen Handstück 1. Das erfindungsgemäße zahnärztliche Handstück 1 weist an seinem instrumentenseitigen und/oder schlauchseitigen Ende eine schräg zur Längsrichtung des zahnärztlichen Handstücks 1 verlaufende Entriegelungsebene oder Entriegelungsschräge 4a bzw. 4b auf. Diese Entriegelungsschräge 4a bzw. 4b ist komplementär zu einer ebenfalls schräg zur Längsrichtung verlaufenden Entriegelungsebene bzw. Entriegelungsschräge 5a bzw. 5b des zahnärztlichen Behandlungsinstruments (Lichtsonde) 2 bzw. des Schlauchanschlusses des in Fig. 1 gezeigten Versorgungschlauches 3 ausgebildet. Die Lichtsonde 2 weist im Kontaktbereich einen Hohlraum 6a auf, der entsprechend zur Außenform des zahnärztlichen Handstücks 1 im Kontaktbereich ausgebildet ist, so daß durch Aufstecken der Lichtsonde 2 auf das zahnärztliche Handstück 1 der instrumentenseitige Bereich des zahnärztlichen Handstücks 1 schlüssig innerhalb des Hohlraumes 6a der Lichtsonde 2 anliegt. Die Entriegelungsschrägen 4a und 5a berühren sich. Die zur Untersuchung eines Zahnes erforderliche Lichtstrahlung wird über den Versorgungsschlauch 3 und einem in dem zahnärztlichen Handstück 1 befindlichen internen Lichtleiter 10 der Lichtsonde 2 zugeführt. Um einen in der Lichtsonde 2 vorhandenen Lichtleiter 11 mit dem in dem zahnärztlichen Handstück 1 angeordneten Lichtleiter 10 zu verbinden, weist die Lichtsonde 2 einen Anschlußbereich 7a auf, der von der Entriegelungsebene 5a der Lichtsonde 2 in das Innere des Hohlraumes 6a hervorsteht. Beim Zusammenstecken der Lichtsonde 2 und des zahnärztlichen Handstückes 1 wird der Anschlußbereich 7a von einer in der schräg verlaufenden Entriegelungsebene 4a ausgebildeten Aufnahmeöffnung 8a aufgenommen, so daß durch Zusammenstecken der beiden Teile die Lichtleiter 11 und 10 miteinander verbunden werden.

Die Funktion des erfindungsgemäßen Kopplungsmechanismusses ist nun wie folgt. Nach Zusammensetzen der Lichtsonde 2 und des zahnärztlichen Handstückes 1 liegt der instrumentenseitige Kopplungsbereich des zahnärztlichen Handstückes 1 schlüssig in dem Hohlraum 6a der Lichtsonde 2a. Die beiden Entriegelungsebenen 4a und 5a berühren sich. Da sowohl die Lichtsonde 2 als auch das zahnärztliche Handstück im Kopplungs- bzw. Kontaktbereich einen kreisförmigen Querschnitt aufweisen, kann die Lichtsonde 2 gegenüber dem zahnärztlichen Handstück 1 in Umfangsrichtung des zahnärztlichen Handstücks 1 verdreht werden. Aufgrund der aneinander anliegenden schräg verlaufenden Entriegungsebenen 4a und 5a wird jedoch unweigerlich durch Verdrehen der Lichtsonde 2 in Umfangsrichtung des zahnärztlichen Handstücks die Lichtsonde 2 in Längsrichtung des zahnärztlichen Handstücks 1 verschoben, d.h. die beiden Teile 1,2 werden durch Verdrehen gegeneinander automatisch getrennt. Da somit erfindungsgemäß zum Trennen der Lichtsonde 2 von dem zahnärztlichen Handstück 1 kein Ziehen der Lichtsonde 2 in Längsrichtung des zahnärztlichen Handstückes 1 von dem Handstück 1 weg mehr erforderlich ist, wird durch den erfindungsgemäßen Entriegelungsmechanismus deutlich der zum Trennen der beiden Teile erforderliche Kraftaufwand reduziert.

Wie bei dem Kopplungs- bzw. Entriegelungsmechanismus zwischen der Lichtsonde 2 und dem zahnärztlichen Handstück 1 weist das zahnärztliche Handstück 1 auch an seinem schlauchseitigen Ende eine Entriegelungsschräge 4b auf, die komplementär zu einer an dem Schlauchanschluß 3 ausgebildeten Entriegelungsschräge 5b ausgebildet ist. Im vorliegenden Fall ist die schlauchseitige Entriegelungsschräge 4b des zahnärztlichen Handstücks 1 in einem Hohlraum 6b des zahnärztlichen Handstücks 1 angeordnet und weist insbesondere eine Aufnahmeöffnung 8b auf, in die ein Anschlußbereich 7b des Schlauchanschlusses 3 eingeführt werden kann. Nach Einführen dieses Anschlußbereiches 7b des Schlauchanschlusses 3 in den Hohlraum 6b des zahnärztlichen Handstückes 1 liegt nicht nur der Anschlußbereich 7b des Versorgungsschlauches 3 schlüssig in der Aufnahmeöffnung 8b des zahnärztlichen Handstückes 1 an, sondern die beiden Entriegelungsebenen 5b und 4b berühren sich insbesondere nach Zusammenstecken des Schlauchanschlusses 3 und des zahnärztlichen Handstückes 1. Da im Kopplungsbereich zwischen dem zahnärztlichen Handstück 1 und dem Versorgungsschlauch 3 beide Teile einen kreisförmigen Querschnitt aufweisen, kann wiederum der Schlauchanschluß 3 gegenüber dem zahnärztlichen Handstück 3 verdreht werden. Wie bereits anhand des Kopplungsmechanismusses zwischen der Lichtsonde 2 und dem zahnärztlichen Handstück 1 beschrieben, wird der Schlauchanschluß 3 durch Verdrehen gegenüber dem zahnärztlichen Handstück 1 in Umfangsrichtung des zahnärztlichen Handstücks 1 aufgrund der aneinander anliegenden Entriegelungsschrägen 4b und 5b automatisch von dem zahnärztlichen Handstück 1 in Längsrichtung des zahnärztlichen Handstücks 1 wegbewegt, so daß ein Trennen der beiden Teile bereits durch Verdrehen der beiden Teile gegeneinander möglich ist, ohne daß der Schlauchanschluß 3 in Längsrichtung des zahnärztlichen Handstück 1 von diesem abgezogen werden muß. Die zum Trennen des Schlauchanschlusses 3 vom zahnärztlichen Handstück 1 erforderliche Kraftanstrengung wird somit deutlich reduziert.

Selbstverständlich kann die in Fig. 2a gezeigte Anordnung auch dahingehend abgewandelt werden, daß die Lichtsonde 2 in einem Hohlraum des zahnärztlichen Handstückes 1 oder das zahnärztliche Handstück 1 in einem Hohlraum des Schlauchanschlusses 3 aufgenommen wird, wobei sich in jedem Fall nach Zusammenstecken der Lichtsonde 2 bzw. des Schlauchanschlusses 3 und des zahnärztlichen Handstückes 1 die Entriegelungsebenen 4a bzw. 4b und 5a bzw. 5b berühren müssen.

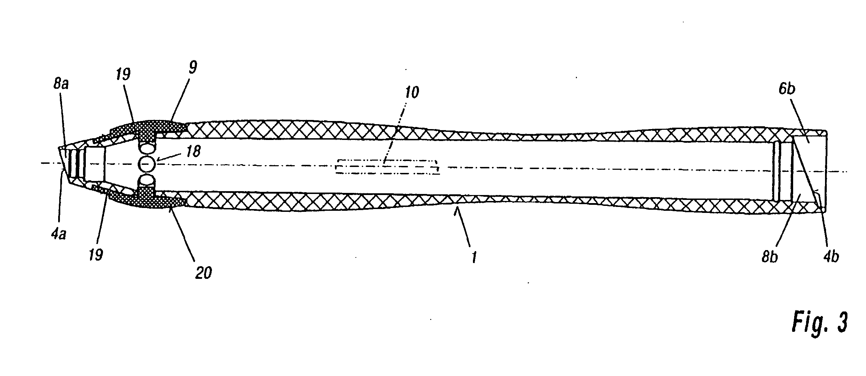

Fig. 3 zeigt eine vergrößerte Darstellung des in Fig. 2a gezeigten zahnärztlichen Handstücks im Querschnitt. Insbesondere sind in Fig. 3 die beiden Entriegelungsebenen 4a und 4b des zahnärztlichen Handstücks 1 sowie die beiden Aufnahmeöffnungen 8a und 8b zur Aufnahme der hervorstehenden Anschlußbereiche 7a und 7b der Lichtsonde 2 bzw. des Schlauchanschlusses 3 dargestellt. Ebenso ist in Fig. 3 näher der Ringschalter 9 dargestellt, der ein ringförmig um das zahnärztliche Handstück 1 herum angeordnetes Betätigungselement 20 umfaßt, welches bei Krafteinwirkung Kontaktmittel 18 aktiviert. Das Betätigungsselement 20 ist durch eine ebenfalls ringförmig um das zahnärztliche Handstück 1 angeordnete Feder 19 elastisch vorgespannt.

Wie bereits anhand von Fig. 1 beschrieben, wird erfindungsgemäß vorgeschlagen, für die Ermittlung von Karies, Plaque oder bakteriellem Befall abhängig von der zu untersuchenden Zahnoberflächengestaltung unteschiedliche Lichtsonden zu verwenden. Insbesondere wird vorgeschlagen, drei unterschiedliche Lichtsonden für die Untersuchung von glatten Zahnoberflächen, Zahnzwischenräumen (Approximalbereichen) oder Fissuren zu verwenden. Fig. 2b - d zeigen jeweils die Spitzen der entsprechenden Lichtsonde für die Untersuchung von glatten Zahnoberflächen (Fig. 2b), Zahnzwischenräumen (Fig. 2c) und Fissuren (Fig. 2d) sowie eine Draufsicht auf die entsprechende Lichtsondenspitze in Pfeilrichtung. Wie aus Fig. 2b - d ersichtlich ist, verläuft die Spitze der Lichtsonde zur Untersuchung glatter Zahnoberflächen eben, während die Lichtsonde zur Untersuchung von Zahnzwischenräumen spitz und die Lichtsonde zur Untersuchung von Fissuren abgerundet verläuft oder kegelstumpfartig ist.

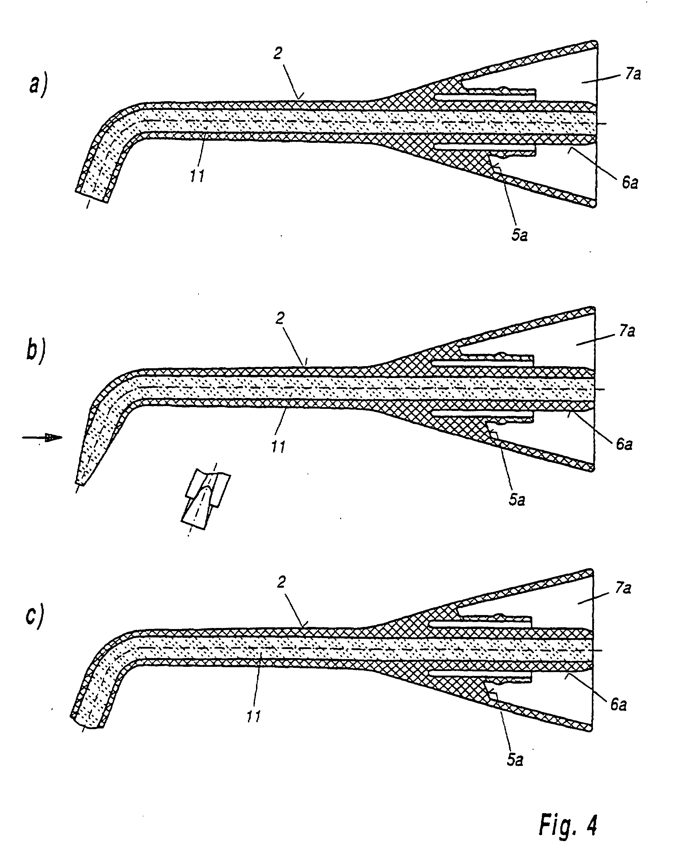

Fig. 4a - c zeigt eine komplette Ansicht der den in Fig. 2b - d gezeigten Lichtsondenspitzen entsprechenden Lichtsonden, wobei insbesondere deutlich die Entriegelungsebene 5a, der Hohlraum 7a zur Aufnahme des instrumentenseitigen Endes des zahnärztlichen Handstücks und der vorspringende Anschlußbereich 6a, der in einer Aufnahmeöffnung des zahnärztlichen Handstücks eingeführt wird, dargestellt sind. Fig. 4b zeigt zudem eine in Pfeilrichtung der Fig. 4b vorgenommene seitliche Draufsicht auf die in Fig. 4b gezeigte Spitze zur Untersuchung von Zahnzwischenräumen.

## Patentansprüche

1. Zahnärztliche Behandlungsvorrichtung,
mit einem zahnärztlichen Handstück (1),
mit einem auf das eine Ende des zahnärztlichen Handstücks aufsteckbaren zahnärztlichen Behandlungsinstrument (2),
mit einem Versorgungsschlauch (3), der mit dem anderen Ende des zahnärztlichen Handstücks zu verbinden ist, um dem zahnärztlichen Handstück die zum Betreiben des zahnärztlichen Behandlungsinstruments erforderlichen Betriebsmittel zuzuführen, und mit einer mit dem Versorgungsschlauch (3) verbundenen Steuereinrichtung (12) zur Steuerung der Zufuhr der Betriebsmittel,
wobei das zahnärztliche Handstück (1) an seinem instrumentenseitigen Ende eine schräg zur Längsrichtung des zahnärztlichen Handstücks verlaufende Entriegelungsebene (4a) aufweist, die komplementär zu einer schräg verlaufenden Entriegelungsebene.(5a) des zahnärztlichen Behandlungsinstruments (2) ausgebildet ist,
wobei im aufgesteckten Zustand das zahnärztliche Behandlungsinstrument (2) durch Paßsitz mit dem zahnärztlichen Handstück (1) fest gekoppelt ist und die Entriegelungsebene (4a) des zahnärztlichen Handstücks (1) an der Entriegelungsebene (5a) des zahnärztlichen Behandlungsinstruments (2) anliegt, und
wobei das zahnärztliche Behandlungsinstrument (2) von dem zahnärztlichen Handstück (1) lediglich durch Verdrehen der beiden Entriegelungsebenen (4a, 5a) gegeneinander in Umfangsrichtung des zahnärztlichen Handstücks (1) entriegelbar ist.

2. Zahnärztliche Behandlungsvorrichtung nach Anspruch 1,
**dadurch gekennzeichnet,**
**daß** das zahnärztliche Handstück (1) an seinem instrumentenseitigen Ende eine Außenform aufweist, die zu einem Hohlraum (6a) des zahnärztlichen Instruments (2) komplementär ausgebildet ist, so daß im zusammengesteckten Zustand das instrumentenseitige Ende des zahnärztlichen Handstücks schlüssig innen in dem Hohlraum des zahnärztlichen Behandlungsinstrumentes anliegt.

3. Zahnärztliche Behandlungsvorrichtung nach Anspruch 1 oder 2,
**dadurch gekennzeichnet,**
**daß** das zahnärztliche Handstück (1) mindestens einen internen Kanal (10) aufweist, um die von dem Versorgungschlauch (3) zugeführten Betriebsmittel dem zahnärztlichen Behandlungsinstrument (2) zuzuführen.

4. Zahnärztliche Behandlungsvorrichtung nach Anspruch 3,
**dadurch gekennzeichnet,**
**daß** das zahnärztliche Behandlungsinstrument (2) einen Anschlußbereich (7a) zur Aufnahme der von dem zahnärztlichen Handstück (1) zugeführten Betriebsmittel aufweist, wobei im zusammengesteckten Zustand der Anschlußbereich des Behandlungsinstruments und der interne Kanal (10) des Handstücks miteinander verbunden sind.

5. Zahnärztliche Behandlungsvorrichfung nach den Ansprüchen 2 und 4,
**dadurch gekennzeichnet,**
**daß** der Anschlußbereich (7a) des zahnärztlichen Behandlungsinstrumentes (2) in das Innere des Hohlraumes (6a) des Behandlungsinstrumentes hervorsteht und im zusammengesteckten Zustand von einer Aufnahmeöffnung (8a) des zahnärztlichen Handstücks (1) aufgenommen wird.

6. Zahnärztliche Behandlungsvorrichtung nach Anspruch 5,
**dadurch gekennzeichnet,**
**daß** die Aufnahmeöffnung (8a) des zahnärztlichen Handstücks (1) in der Entriegelungsebene (4a) des Handstücks ausgebildet ist und der Anschlußbereich (7a) des zahnärztlichen Behandlungsinstrumentes (2) von der Entriegelungsebene (5a) des Behandlungsinstrumentes hervorsteht:

7. Zahnärztliche Behandlungsvorrichtung nach Anspruch 1,
**dadurch gekennzeichnet,**
**daß** das zahnärztliche Behandlungsinstrument (2) an seinem handstückseitigen Ende eine Außenform aufweist, die zu einem Hohlraum des zahnärztlichen Handstücks (1) komplementär ausgebildet ist, so daß im zusammengesteckten Zustand das handstückseitige Ende des zahnärztlichen Behandlungsinstruments schlüssig innen in dem Hohlraum des zahnärztlichen Handstücks anliegt.

8. Zahnärztliche Behandlungsvorrichtung nach einem der vorherigen.Ansprüche,
**dadurch gekennzeichnet,**
**dass** ein handstückseitiger Schlauchanschluß des Versorgungsschlauches (3) eine zur Längsrichtung des Schlauchanschlusses schräg verlaufende Entriegelungsebene (5b) aufweist, die komplementär zu einer weiteren schräg verlaufenden Entriegelungsebene (4b) des zahnärztlichen Handstücks (1) ausgebildet ist,
wobei im aufgesteckten Zustand der Versorgungsschlauch (3) durch Paßsitz mit dem zahnärztlichen Handstück (1) fest gekoppelt ist und die weitere Entriegelungsebene (4b) des zahnärztlichen Handstücks (1) an der Entriegelungsebene (5b) des Versörgungsschlauchs (3) anliegt, und
wobei der Versorgungsschlauch (3) von dem zahnärztlichen Handstück (1) lediglich durch Verdrehen dieser beiden Entriegelungsebenen (4b, 5b) gegeneinander in Umfangsrichtung des zahnärztlichen Handstücks (1) entriegelbar ist.

9. Zahnärztliche Behandlungsvorrichtüng nach Anspruch 8,
**dadurch gekennzeichnet,**
**daß** der Schlauchanschluß des Versorgungsschlauches (3) an seinem handstückseitigen Ende eine Außenform aufweist, die zu einem Hohlraum (6b) des zahnärztlichen Handstücks (1) komplementär ausgebildet ist, so daß im zusammengestecken Zustand das handstückseitige Ende des Schlauchanschlusses schlüssig innen in dem Hohlraum (6b) des zahnärztlichen Handstücks (1) anliegt.

10. Zahnärztliche Behandlungsvorrichtung nach Anspruch 9,
**dadurch gekennzeichnet,**
**daß** der Schlauchanschluß (3) einen von der Entriegelungsebene (5b) des Schlauchanschlusses hervorstehenden Anschlußbereich (7b) aufweist, der im zusammengesteckten Zustand von einer in der weiteren Entriegelungsebene (4b) des zahnärztlichen Handstücks (1) ausgebildeten Aufnahmeöffnung (8b) des Hohlraumes (6b) aufgenommen wird.

11. Zahnärztliche Behandlungsvorrichtung nach Anspruch 8,
**dadurch gekennzeichnet,**
**daß** das zahnärztliche Handstück (1) an seinem schlauchseitigen Ende eine Außenform aufweist, die zu einem Hohlraum des Schlauchanschlusses des Versorgungsschlauches (3) komplementär ausgebildet ist, so daß im zusammengesteckten Zustand das schlauchseitige Ende des zahnärztlichen Handstücks (1) schlüssig innen in dem Hohlraum des Schlauchanschlusses anliegt.

12. Zahnärztliche Behandlungsvorrichtung nach einem der vorhergehenden Ansprüche,
**dadurch gekennzeichnet,**
**daß** das zahnärztliche Behandlungsinstrument (2) eine abnehmbare Lichtsonde ist, welche im zusammengesteckten Zustand über Lichtleiter (10) mit der Steuereinrichtung (12) verbunden ist, und
**daß** die zahnärztliche Behandlungsvorrichtung zum Ermitteln von Karies, Plaque oder bakteriellem Befall von Zähnen dienen kann, wobei die Lichtsonde (2). die Zähne mit einer Primär-Lichtstrahlung bestrahlen und die somit an den bestrahlten, Zähnen angeregte Sekundär-Fluoreszenzstrahlung detektieren kann und die Steuereinrichtung die Sekundär-Fluoreszenzstrahlung über die Lichtleiter (10) empfangen und auswerfen kann.

13. Zahnärztliche Behandlungsvorrichtung nach Anspruch 12,
**dadurch gekennzeichnet,**
**daß** an das zahnärztliche Handstück (1) abhängig von dem Verwendungszweck eine Vielzahl unterschiedlicher Lichtsonden (2) anschließbar ist.

14. Zahnärztliche Behandlungsvorrichtung nach Anspruch 13,
**dadurch gekennzeichnet,**
**daß** eine Lichtsonde für die Untersuchung von glatten Zahnoberflächen, eine Lichtsonde; für die Untersuchung von Zwischenzahnbereichen und eine Lichtsonde für die Untersuchung von Fissuren vorgesehen ist.

15. Zahnärztliche Behandlungsvorrichtung nach Anspruch 14,
**dadurch gekennzeichnet,**
**daß** die Lichtsonde (2) für die Untersuchung von glatten Zahnoberflächen an ihrem zahnseitigen Ende flach läuft.

16. Zahnärztliche Behandlungsvorrichtung nach Anspruch 14 oder 15,
**dadurch gekennzeichnet,**
**daß** die Lichtsonde (2) für die Untersuchung von Zwischenzahnbereichen an ihrem zahnseitigen Ende spitz ist.

17. Zahnärztliche Behandlungsvorrichtung nach einem der Ansprüche 14 bis 16,
**dadurch gekennzeichnet,**
**daß** die Lichtsonde (2) für die Untersuchung von Fissuren an ihrem zahnseitigen Ende abgerundet oder kegelstumpfartig ist.

18. Zahnärztliche Behandlungsvorrichtung nach einem der Ansprüche, 13 bis 17,
**dadurch gekennzeichnet,**
**daß** eine Anzeige (15a-c) zum Anzeigen der auf das zahnärztliche Handstück (1) aufgesteckten Lichtsonde (2) vorgesehen ist.

19. Zahnärztliche Behandlungsvorrichtung nach einem der Ansprüche: 13 bis 18,
**dadurch gekennzeichnet,**
**daß** Mittel (17) vorgesehen sind, um der Steuereinrichtung (12) den Typ der auf das zahnärztliche Handstück (1) aufgesteckten Lichtsonde (2) mitzuteilen.

## Claims

1. Dental treatment device,
having a dental handpiece (1),
having a dental treatment instrument (2), which can be plugged on to the one end of the dental handpiece,
having a supply hose (3), which can be connected to a the other end of the dental handpiece, in order to deliver the operating medium needed for the operation of the dental treatment instrument, and
having a control device (12) connected with the supply hose (3) for controlling the delivery of the operating medium,
wherein the dental handpiece (1) has at its instrument end an unlocking plane (4a) running obliquely to the longitudinal direction of the dental handpiece, which plane is formed complementary to an obliquely running unlocking plane (5a) of the dental treatment instrument (2),
wherein in the plugged-on condition, the dental treatment instrument (2) is firmly coupled with the dental handpiece (1) by means of a press fit and the unlocking plane (4a) of the dental handpiece (1) bears on the unlocking plane (5a) of the dental treatment instrument (2), and
wherein the dental treatment instrument (2) can be unlocked from the dental handpiece (1) solely by turning of the two unlocking planes (4a, 5a) against one another in the circumferential direction of the dental handpiece (1).

2. Dental treatment device according to claim 1,
**characterized in that**,
the dental handpiece (1) has at its instrument end an external form which is constituted complementary to a hollow space (6a) of the dental treatment instrument (2), so that in the plugged-together condition the instrument end of the dental handpiece bears in a fitted manner internally in the hollow space of the dental treatment instrument.

3. Dental treatment device according to claim 1 or 2,
**characterized in that**,
the dental handpiece (1) has at least one internal channel (10) in order to deliver to the dental treatment instrument (2) the operating medium delivered from the supply hose (3).

4. Dental treatment device according to claim 3,
**characterized in that**,
the dental treatment instrument (2) has a connection region (7a) for receiving the operating medium delivered from the dental handpiece (1), whereby in the plugged-together condition the connection region of the treatment instrument and the internal channel (10) of the handpiece are connected with one another.

5. Dental treatment device according to claims 2 and 4,
**characterized in that**,
the connection region (7a) of the dental treatment instrument (2) projects into the interior of the hollow space (6a) of the treatment instrument and in the plugged-together condition is received by a receiving opening (8a) of the dental handpiece (1).

6. Dental treatment device according to claim 5,
**characterized in that**,
the receiving opening (8a) of the dental handpiece (1) is formed in the unlocking plane (4a) of the handpiece and the connection region (7a) of the dental treatment instrument (2) projects from the unlocking plane (5a) of the treatment instrument.

7. Dental treatment device according to claim 1,
**characterized in that**,
the dental treatment instrument (2) has at its handpiece end an exterior form which is constituted complementary to a hollow space of the dental handpiece (1), so that in the plugged-together condition the handpiece end of the dental treatment instrument bears in a fitted manner internally in the hollow space of the dental handpiece.

8. Dental treatment instrument according to any preceding claim,
**characterized in that**,
a handpiece-end hose connection of the supply hose (3) has an unlocking plane (5b) running obliquely to the longitudinal direction of the hose connection, which plane is formed complementary to a further obliquely running unlocking plane (4b) of the dental handpiece (1),
wherein in the plugged-on condition, the supply hose (3) is firmly coupled with the dental handpiece (1) by means of a press fit and the further unlocking plane (4b) of the dental handpiece (1) bears on the unlocking plane (5b) of the supply hose (3), and
wherein the supply hose (3) can be unlocked from the dental handpiece (1) solely by turning these two unlocking planes against one another in the circumferential direction of the dental handpiece (1).

9. Dental treatment device according to claim 8,
**characterized in that**,
the hose connection of the supply hose (3) has at its handpiece end an exterior form which is constituted complementary to a hollow space (6b) of the dental handpiece (1), so that in the plugged-together condition the handpiece end of the hose connection bears in a fitted manner internally in the hollow space (6b) of the dental handpiece (1).

10. Dental treatment device according to claim 9,
**characterized in that**,
the hose connection (3) has a connection region (7b) projecting from the unlocking plane (5b) of the hose connection, which connection region is, in the plugged-together condition, received by a receiving opening (8b) of the hollow space (6b), formed in the further unlocking plane (4b) of the dental handpiece (1).

11. Dental treatment device according to claim 8,
**characterized in that**,
the dental handpiece (1) has at its hose end an exterior form which is constituted complementary to a hollow space of the hose connection of the supply hose (3), so that in the plugged together condition the hose end of the dental handpiece (1) bears in a fitted manner internally in the hollow space of the hose connection.

12. Dental treatment device according to any preceding claim,
**characterized in that**,
the dental treatment instrument (2) is a removable light probe, which in the plugged-together condition is connected with the control device (12) via light conductors (10), and
**in that** the dental treatment device can serve for the detection of caries, plaque or bacterial infection of teeth, whereby the light probe (2) irradiates the teeth with primary light radiation and detects secondary fluorescence radiation thus excited at the irradiated teeth, and the control device can receive and evaluate the secondary fluorescence radiation via the light conductor (10).

13. Dental treatment device according to claim 15,
**characterized in that**,
the dental handpiece (1) can be connected to a plurality of different light probes (2) in dependence upon the intended use.

14. Dental treatment device according to claim 13,
**characterized in that**,
there is provided a light probe for the investigation of smooth tooth surfaces, a light probe for the investigation of regions between the teeth, and a light probe for the investigation of fissures.

15. Dental treatment device according to claim 14,
**characterized in that**,
the light probe (2) for the investigation of smooth tooth surfaces is flat at its end towards the teeth.

16. Dental treatment device according to claim 14 or 15,
**characterized in that**,
the light probe (2) for the investigation of regions between the teeth is pointed at its end towards the teeth.

17. Dental treatment device according to any of claims 14 to 16,
**characterized in that**,
the light probe (2) for the investigation of fissures is rounded or truncated cone shape at its end towards the teeth.

18. Dental treatment device according to any of claims 13 to 17,
**characterized in that**,
a display (15a-c) is provided for indicating the light probe (2) plugged on to the dental handpiece (1).

19. Dental treatment device according to any of claims 13 to 18,
**characterized in that**,
means (17) are provided in order to indicate to the control device (12) the type of light probe (2) plugged on to the dental handpiece (1).

## Revendications

1. Dispositif de traitement dentaire
comprenant une poignée (1) de soins dentaires,
un instrument (2) de soins dentaires, pouvant être emboîté sur l'une des extrémités de ladite poignée de soins dentaires,
un flexible d'alimentation (3) conçu pour être relié à l'autre extrémité de la poignée de soins dentaires afin de délivrer, à ladite poignée de soins dentaires, les agents de fonctionnement nécessaires à l'actionnement de l'instrument de soins dentaires, et
un système de commande (12) relié au flexible d'alimentation (3), en vue de commander la délivrance des agents de fonctionnement,
sachant que la poignée (1) de soins dentaires présente, à son extrémité située côté instrument, un plan de déverrouillage (4a) s'étendant à l'oblique par rapport à la direction longitudinale de ladite poignée de soins dentaires, et dont la réalisation est complémentaire de celle d'un plan de déverrouillage (5a) de l'instrument (2) de soins dentaires, s'étendant à l'oblique ;
sachant que, à l'état emboîté, l'instrument (2) de soins dentaires est fermement accouplé par ajustement serré avec la poignée (1) de soins dentaires, et le plan de déverrouillage (4a) de ladite poignée (1) de soins dentaires est appliqué contre le plan de déverrouillage (5a) dudit instrument (2) de soins dentaires ; et
sachant que l'instrument (2) de soins dentaires peut être déverrouillé, d'avec la poignée (1) de soins dentaires, uniquement par rotation des deux plans de déverrouillage (4a, 5a), l'un par rapport à l'autre, dans la direction périphérique de ladite poignée (1) de soins dentaires.

2. Dispositif de traitement dentaire selon la revendication 1,
**caractérisé par le fait**
**que** la poignée (1) de soins dentaires possède, à son extrémité située côté instrument, une configuration extérieure de réalisation complémentaire de celle d'une cavité (6a) de l'instrument (2) de soins dentaires, de telle sorte que, à l'état emboîté, l'extrémité de ladite poignée de soins dentaires, qui est située côté instrument, soit logée intimement à l'intérieur de ladite cavité dudit instrument de soins dentaires.

3. Dispositif de traitement dentaire selon la revendication 1 ou 2,
**caractérisé par le fait**
**que** la poignée (1) de soins dentaires présente au moins un canal intérieur (10) en vue de diriger, vers l'instrument (2) de soins dentaires, les agents de fonctionnement délivrés par le flexible d'alimentation (3).

4. Dispositif de traitement dentaire selon la revendication 3,
**caractérisé par le fait**
**que** l'instrument (2) de soins dentaires présente une zone de raccordement (7a) destinée à recevoir les agents de fonctionnement délivrés par la poignée (1) de soins dentaires, sachant que la zone de raccordement de l'instrument de soins dentaires et le canal intérieur (10) de la poignée sont reliés l'un à l'autre à l'état solidarisé par emboîtement.

5. Dispositif de traitement dentaire selon les revendications 2 et 4,
**caractérisé par le fait**
**que** la zone de raccordement (7a) de l'instrument (2) de soins dentaires fait saillie dans l'espace interne de la cavité (6a) dudit instrument de soins et est reçue, à l'état solidarisé par emboîtement, par un orifice récepteur (8a) de la poignée (1) de soins dentaires.

6. Dispositif de traitement dentaire selon la revendication 5,
**caractérisé par le fait**
**que** l'orifice récepteur (8a) de la poignée (1) de soins dentaires est façonné dans le plan de déverrouillage (4a) de ladite poignée, et la zone de raccordement (7a) de l'instrument (2) de soins dentaires dépasse au-delà du plan de déverrouillage (5a) dudit instrument de soins.

7. Dispositif de traitement dentaire selon la revendication 1,
**caractérisé par le fait**
**que** l'instrument (2) de soins dentaires possède, à son extrémité située côté poignée, une configuration extérieure de réalisation complémentaire de celle d'une cavité de la poignée (1) de soins dentaires, de façon telle que, à l'état solidarisé par emboîtement, l'extrémité dudit instrument de soins dentaires, qui est située côté poignée, soit intimement logée à l'intérieur de ladite cavité de ladite poignée de soins dentaires.

8. Dispositif de traitement dentaire selon l'une des revendications précédentes,
**caractérisé par le fait**
**qu'**un raccord du flexible d'alimentation (3), situé côté poignée, présente un plan de déverrouillage (5b) qui s'étend à l'oblique par rapport à la direction longitudinale dudit raccord du flexible, et dont la réalisation est complémentaire de celle d'un autre plan de déverrouillage (4b) de la poignée (1) de soins dentaires, s'étendant à l'oblique ;
sachant que, à l'état emboîté, le flexible d'alimentation (3) est fermement accouplé par ajustement serré avec la poignée (1) de soins dentaires, et le plan supplémentaire de déverrouillage (4b) de ladite poignée (1) de soins dentaires est appliqué contre le plan de déverrouillage (5b) dudit flexible d'alimentation (3) ; et
sachant que ledit flexible d'alimentation (3) peut être déverrouillé, d'avec ladite poignée (1) de soins dentaires, uniquement par rotation de ces deux plans de déverrouillage (4b, 5b), l'un par rapport à l'autre, dans la direction longitudinale de ladite poignée (1) de soins dentaires.

9. Dispositif de traitement dentaire selon la revendication 8,
**caractérisé par le fait**
**que** le raccord du flexible d'alimentation (3) possède, à son extrémité située côté poignée, une configuration extérieure dont la réalisation est complémentaire de celle d'une cavité (6b) de la poignée (1) de soins dentaires, de façon telle que, à l'état solidarisé par emboîtement, l'extrémité dudit raccord du flexible, qui est située côté poignée, soit logée intimement à l'intérieur de ladite cavité (6b) de ladite poignée (1) de soins dentaires.

10. Dispositif de traitement dentaire selon la revendication 9,
**caractérisé par le fait**
**que** le raccord du flexible (3) présente une zone de raccordement (7b) qui dépasse au-delà du plan de déverrouillage (5b) dudit raccord du flexible et qui, à l'état solidarisé par emboîtement, est reçue par un orifice récepteur (8b) de la cavité (6b), façonné dans le plan supplémentaire de déverrouillage (4b) de la poignée (1) de soins dentaires.

11. Dispositif de traitement dentaire selon la revendication 8,
**caractérisé par le fait**
**que** la poignée (1) de soins dentaires possède, à son extrémité située côté flexible, une configuration extérieure dont la réalisation est complémentaire de celle d'une cavité du raccord du flexible d'alimentation (3), de façon telle que, à l'état solidarisé par emboîtement, l'extrémité de ladite poignée (1) de soins dentaires, située côté flexible, soit logée intimement à l'intérieur de ladite cavité dudit raccord du flexible.

12. Dispositif de traitement dentaire selon l'une des revendications précédentes,
**caractérisé par le fait**
**que** l'instrument (2) de soins dentaires est une sonde lumineuse amovible qui, à l'état solidarisé par emboîtement, est reliée au système de commande (12) par l'intermédiaire de guides de lumière (10) ; et
par le fait que ledit dispositif de traitement dentaire peut servir à déterminer des caries, une plaque ou une infestation bactérienne des dents, sachant que la sonde lumineuse (2) peut bombarder les dents par un rayonnement de lumière primaire, et détecter le rayonnement de fluorescence secondaire ainsi provoqué sur les dents bombardées ; et que le système de commande peut recevoir ledit rayonnement de fluorescence secondaire, par l'intermédiaire des guides de lumière (10), et interpréter ledit rayonnement.

13. Dispositif de traitement dentaire selon la revendication 12,
**caractérisé par le fait**
**qu'**une multiplicité de sondes lumineuses différentes (2) peut être raccordée à la poignée (1) de soins dentaires, en fonction de l'objectif d'utilisation.

14. Dispositif de traitement dentaire selon la revendication 13,
**caractérisé par le fait**
**que** sont prévues une sonde lumineuse destinée à l'examen de surfaces dentaires lisses, une sonde lumineuse destinée à l'examen d'espaces interstitiels dentaires, et une sonde lumineuse destinée à l'examen de fissures.

15. Dispositif de traitement dentaire selon la revendication 14,
**caractérisé par le fait**
**que** la sonde lumineuse (2), affectée à l'examen de surfaces dentaires lisses, présente une étendue plate à son extrémité située côté dents.

16. Dispositif de traitement dentaire selon la revendication 14 ou 15,
**caractérisé par le fait**
**que** la sonde lumineuse (2), affectée à l'examen d'espaces interstitiels dentaires, est pointue à son extrémité située côté dents.

17. Dispositif de traitement dentaire selon l'une des revendications 14 à 16,
**caractérisé par le fait**
**que** la sonde lumineuse (2), affectée à l'examen de fissures, est arrondie ou tronconique à son extrémité située côté dents.

18. Dispositif de traitement dentaire selon l'une des revendications 13 à 17,
**caractérisé par le fait**
**qu'**un affichage (15a-c) est prévu pour indiquer la présence de la sonde lumineuse (2) emboîtée sur la poignée (1) de soins dentaires.

19. Dispositif de traitement dentaire selon l'une des revendications 13 à 18,
**caractérisé par le fait**
**que** des moyens (17) sont prévus pour indiquer, au système de commande (12), le type de la sonde lumineuse (2) emboîtée sur la poignée (1) de soins dentaires.
